Europäisches Patentamt

European Patent Office

Office européen des brevets

(11) Publication number: **0 219 133**
**A1**

## (12) EUROPEAN PATENT APPLICATION

(21) Application number: **86114363.4**

(22) Date of filing: **16.10.86**

(51) Int. Cl.⁴: **A01G 7/00 , A01G 31/00**

(30) Priority: **17.10.85 US 788330**

(43) Date of publication of application:
**22.04.87 Bulletin 87/17**

(84) Designated Contracting States:
**AT BE CH DE ES FR GB GR IT LI LU NL SE**

(71) Applicant: **DNA PLANT TECHNOLOGY
CORPORATION (under the laws of the state
of Delaware)
2611 Branch Pike
Cinnaminson, New Jersey(US)**

(72) Inventor: **Styer, Donald J.
6313 Wyndam Road
Pennsauken New Jersey(US)**

(74) Representative: **Patentanwälte Grünecker,
Kinkeldey, Stockmair & Partner
Maximilianstrasse 58
D-8000 München 22(DE)**

(54) **A process and apparatus for growing plantlets from embryos.**

(57) A process and apparatus for growing plantlets from embryos. The process includes the steps of providing a sterile growth environment including reticulated foam, a culture medium, and a container holding the foam and the culture medium; inoculating the foam with the embryos; and incubating the embryos under sterile, plantlet development conditions to develop plantlets from the embryos. Once the plantlets have developed, they may be removed from the growth environment; and this may be done by removing the foam and plantlets from the container, removing any remaining culture medium from the foam, and covering the foam and the roots of the plantlets with a potting mix.

FIG.I

# A PROCESS AND APPARATUS FOR GROWING PLANTLETS FROM EMBRYOS

This invention generally relates to a process and apparatus for growing plantlets from embryos, and more particularly to a process, and to apparatus for carrying out that process, that may be used to grow plantlets from somatic embryos, and that are well suited for use on a highly automated, large scale basis.

A tremendous number of plants are grown commercially each year. For instance, it is necessary to replant at least one billion pine trees to provide adequate reforestation in the United States of America alone; and huge quantities of other plants such as flowers and vegetables, are needed every year for agricultural, environmental and decorative purposes. At present, many plants are grown by very expensive, labor intensive methods; and while numerous large scale, automated plant growing processes have been tried, these processes have met with only limited success, either because they are expensive or because it is difficult to control satisfactorily the quality of the grown plants.

The difficulties associated with growing plants on a large scale, highly automated basis, are exacerbated when the plants are grown from somatic embryos, as opposed to seeds, since somatic embryos do not have the same natural nutrient supply and physical protection that seeds have. At the same time, growing plants from somatic embryos has significant advantages, first, because it is sometimes difficult to obtain seeds from plants grown in areas that are relatively inaccessible, either on account of the terrain, weather or for other reasons; and second, because very desirable traits, such as resistance to particular diseases or a preferred fruit strain, may be duplicated exactly in plants grown from somatic embryos, while such duplication cannot normally be guaranteed, and in fact may be very difficult to obtain, in plants grown from seeds.

Various techniques are known for growing plant cells and tissues; however, most, if not all, of these techniques are not well suited for growing plantlets from embryos on a large scale commercial basis because the needs of plantlets and embryos differ substantially from the needs of cells and tissues, and also because plantlets and embryos are physically very different from plant cell and tissue cultures. For example, to grow large numbers of plantlets economically, it is important to prevent the plantlets from becoming tangled with each other because untangling the plantlets is a very time consuming and costly process, and similar problems are not normally encountered when growing cell and tissue cultures. Further, in order to grow plantlets on a practical, large scale basis, it is important to be able to handle, move and transplant the plantlets in a comparatively simple and inexpensive manner, while these considerations usually do not have similar importance when growing plant cells and tissues.

The present invention relates to a process for growing plantlets from embryos comprising the steps of providing a sterile growth environment including reticulated foam, a culture medium, and a container holding the foam and the culture medium; inoculating the foam with the embryos; and incubating the embryos under sterile plantlet development conditions to grow plantlets from the embryos. Once the plantlets have developed, they may be removed from the growth environment; and this may be done by removing the foam and plantlets from the container, removing any remaining culture medium from the foam, and covering the foam and the roots of the plantlets with a granular plant growth and support material such as a potting mix or soil.

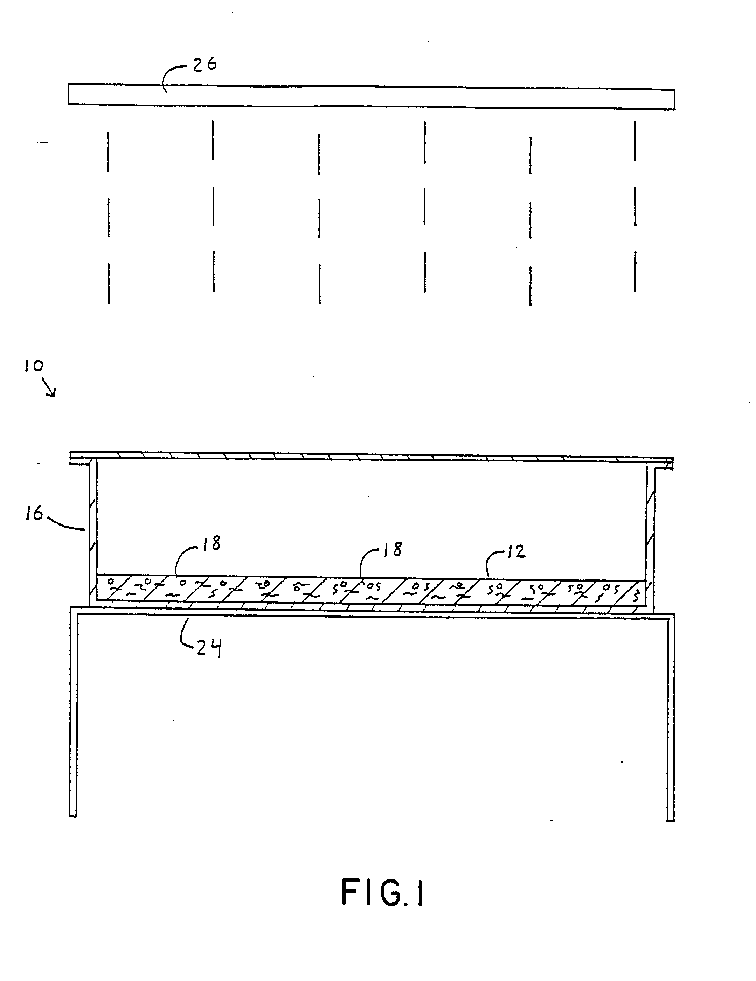

The sole Figure in the drawing is a simplified cross-sectional view of apparatus for growing plantlets from embryos in accordance with this invention.

In accordance with the present invention, it has been learned that plantlets may be grown from embryos, and in particular somatic embryos, on a reticulated foam. To do this, with reference to the accompanying drawing, a sterile growth environment 10 is provided including the foam 12, a culture medium, and a container 16 holding the foam and the culture medium. The foam is inoculated with the embryos 18, and then the embryos are incubated under sterile plantlet development conditions to grow plantlets from the embryos. Once the plantlets have developed, they may be removed from the growth environment; and this may be done by removing the foam and the plantlets from the container, removing any remaining culture medium from the foam, and covering the foam and the roots of the plantlets with a suitable granular plant growth and support material.

Preferably, the sterile growth environment 10 is provided by placing the reticulated foam 12 in the container 16, sterilizing the container and the foam, and then adding a sterile culture medium to the foam. With an embodiment of the invention that has been actually reduced to practice, the culture medium is a liquid and is added to the foam prior to inoculating the foam with the embryos 18, and the container is closed after the foam is inoculated with the embryos to keep the embryos sterile and to reduce evaporation of the culture medium.

Many types of containers 16 may be used in this invention. It is important, first, that the container be capable of being sterilized, and second, that light be able to pass into the interior of the container when it is closed. For example, shallow aluminum pans or transparent polycarbonate cages, covered with sheets of polyvinyl chloride film, may be used to hold the foam, the culture medium and plantlets. Aluminum pans have the advantage that they are very inexpensive, while polycarbonate cages have the advantage that they readily pass light into the interior of the containers. It should be noted that a part, and even a substantial part, of the container may be opaque, and it is not necessary that any part of the container be transparent. At the same time, since light generally promotes plant growth, it is normally preferred that a substantial portion of the container be transparent.

When the container 16 is closed, it is desirable that the container be sufficiently sealed so that microbes do not pass into the interior of the container. Furthermore, it has been found beneficial to use a container cover, such as a polyvinyl chloride film, that is gas permeable so that water vapor, carbon dioxide and oxygen can diffuse through the cover, into or out from the interior of the container, as the embryos 18 are incubated and the plantlets grow from those embryos.

Similarly, any suitable reticulated foam 12 may be used in the practice of this invention. Preferably, the foam has a large percentage of open or connected cells; however, it is not necessary that all or even a majority of the cells be open. The specific size and shape of the cells and the particular density of the foam are not believed to be critical to the effective operation of this invention. The cells should be large enough so that the culture medium spreads thoroughly throughout the interior of the foam, yet small enough so that the culture medium is held within the interior of the foam and does not simply pass completely through the foam. Also, the cells should be small enough so that the embryos do not fall through the foam, yet large enough to permit roots from the embryos to grow in and through the cells. Preferably, the foam has sufficient strength to maintain its integrity when wet, and may be handled without breaking apart so that the plantlets may be removed from the container, transported and transplanted by handling only the foam structure and without grasping the plantlets themselves. It is further desirable that the foam be biodegradable so that the foam may decompose naturally after being transplanted.

With a preferred embodiment of this invention, the foam 12 is cut into a multitude of small foam squares, and these squares are used to line the bottom of the container. The specific dimensions of these small foam squares are not critical, nor is it critical that the squares all be of the same size. With one group of plantlets grown in accordance with this invention, the foam squares had widths and lengths of about one inch, and depths of about one-half inch.

The container 16 and the foam 12 may be sterilized in any acceptable manner. For instance, the container and the foam can be steam autoclaved under pressure. It is preferred, but not essential, that the container and foam be sterilized together; but, alternately, the foam can be placed in the container after they have both been sterilized separately. The former arrangement is preferred because it eliminates the possibility that the handling of the foam necessary to place it in the container may contaminate the foam or container.

Many types of plant culture media may be added to the foam to support the embryo growth. Such media must provide the macronutrients essential to plant growth: calcium, nitrogen, phosphorus, sulfur, potassium, magnesium and sucrose, glucose or other sources of energy and carbon; and preferably the media also include the micronutrients or trace elements: boron, manganese, iron, zinc, molybdenum and copper which are necessary for intensive plant growth. As previously mentioned, preferably the culture medium is in the form of a liquid, with the above-listed ingredients being in solution in that liquid, and the culture medium is added to the foam by simply pouring or spraying the liquid culture medium onto the foam.

In order to obtain the best results, the foam 12 is thoroughly soaked with the culture medium to insure that a sufficient supply of that medium is available for consumption, first, by the embryos 18 and, later, by the plantlets over an extended period of time. As indicated above, preferably the culture medium is added to the foam after the foam and container are sterilized, and the culture medium is sterile when added to the foam. However, the culture medium may be added to the foam either before or after the container and the foam are sterilized; and if the medium is added to the foam before the foam and container are sterilized, it is not necessary that the culture medium be sterile when it is added to the foam since the medium can be sterilized with the foam and the container. As a practical matter, though, it is felt to be easier to sterilize the culture medium separately from the foam and the container, and to add the sterile culture medium to the foam after the foam and the container have themselves both been sterilized.

The foam 12 may be inoculated with the embryos 18 in various ways. For example, the embryos may be aspirated into a pipette or dropper from a flask or beaker holding a nutrient solution containing the embryos, and then dispensed from the pipette or dropper onto or into the reticulated foam. Preferably, the embryos are inoculated directly into the interior of the foam, and it is believed to be advantageous, although not necessary, to keep the embryos in the upper portion of the foam so that foam does not shade the embryos from too much light.

It is also desirable to distribute the embryos 18 generally uniformly around the foam 12 to help insure an adequate supply of culture medium for each embryo in the immediate vicinity of the embryo and also to help prevent the plantlets from becoming tangled with each other as they grow from the embryos. With the preferred embodiment of this invention, where the foam used to support the embryos is comprised of a multitude of small foam squares, preferably each foam square is inoculated with only one or at most a few embryos to keep the amount of the culture medium per embryo relatively high for each embryo. Ideally, a single embryo is deposited in an upper central portion of each foam square, although as a practical matter this is very difficult to do, and is not an essential part of this invention.

It is desirable to inoculate the foam 12 with the embryos 18 after the culture medium has been added to that foam because, if the foam is inoculated with the embryos first, the embryos may be washed or splashed from the foam as the culture medium is added. The present invention has been successfully used to grow plantlets from embryos deposited on the foam at various stages of embryo development, and it is believed that the more mature the embryo is when it is inoculated into or onto the foam, the better the embryo's chance for surviving and growing on the foam.

The embryos 18 may be incubated in any acceptable manner. It is important that the embryos receive adequate light; and one procedure that has been successfully used is to place the container 16, with the foam 12 and the embryos 18 on the bottom thereof, on racks 24 located about 14 inches below a 40 watt fluorescent light bulb 26, and to alternately turn the light on for 16 hours and then off for 8 hours. Embryos have also been successfully grown with continuous light. In addition, it is preferred that the temperature of the embryos normally be maintained about room temperature, 22°C, although it is believed that satisfactory results may be obtained as long as the embryos are usually kept between about 15°C and 30°C. Preferably, the container is normally kept closed during the incubation period, although it may be occasionally opened to change the culture medium or to inspect the embryos and plantlets visually, for example.

As the embryos 18 are incubated, they develop into small plantlets, with roots growing through the cells of the foam 12 and with leaves developing on the stalks or stems of the plantlets. As these roots grow, they reach into and through different cells of the foam, and it is believed that an important aspect of this invention is that as the culture medium in a particular cell is consumed by an embryo, the roots of that embryo grow into another cell and come into contact with the culture medium in that other cell, replenishing the nutrient supply for the embryo.

The plantlets may be removed from the container 16 at any time, but it is felt best to remove the plantlets when they have two or three leaves. To elaborate, leaves produce essential nutrients for plants, and the more leaves a transplanted plant has, the better its chances for survival. Normally, a transplanted plant with only one leaf does not have a very good chance of surviving, while a plant with two or three leaves has a very good chance of surviving. At the same time, the leaves that are on a plant when it is transplanted usually drop off the plant soon after it is transplanted, resulting in a bare, unproductive area on the plant. Transplanting a plantlet with two or three leaves is a very effective compromise between the objective of maximizing the plants chances for surviving the transplantation and the object of minimizing the bare unproductive area on the plant caused by leaves falling off the plant after transplantation. It has been found that the desirable plantlet development can be obtained during incubation periods of between approximately 12 and 20 days.

Much of the culture medium added to the foam 12 is consumed by the embryos 18 and plantlets as they grow in the foam; and any culture medium remaining in the foam when it and the plantlets are taken out of the container 16, is then removed from the foam since the culture medium may support microbial growth once that medium is removed from the sterilized container. The culture medium may be removed in any suitable way, for example by spraying or pouring water through the foam, though care should be taken not to damage the plantlets or their roots or leaves.

After the foam and plantlets are removed from the container and any remaining culture medium is removed from the foam, the foam and the plantlets may be transplanted in another location. Preferably, when so transplanted, the foam, the roots and the lower portions of the plantlets, up to the middle of the hypocotyle, are covered with a fine grain plant growth and support material. This fine grain material may be, or include, soil, vermiculite, peat moss, perlite, similar substances, or mixtures thereof, and many suitable potting mixes are readily available and may be used in the practice of this invention.

It may be advantageous to condition the plantlets to that other location before they are planted therein. More specifically, with the above-described growing process, a relatively high humidity develops inside the container as the plantlets grow, and it is desirable to accustom the plantlets to a more normal humidity before they are transplanted. As will be appreciated, this may be done in several ways. With one procedure, the foam and plantlets are removed from the container in which they are originally grown and placed in a second container; and the foam, the roots and the lower portions of the plantlets are covered with a potting mix. This second container is itself covered with a plastic film and placed either in the location where the plantlets will be transplanted, or in an area having a humidity approximately equal to the humidity of that location. After allowing the plantlets a few days to overcome the shock of the transplantation into this second container, a small hole is punched or otherwise formed in the cover of the second container. With this small hole, the humidity inside the container gradually decreases until an equilibrium is reached between the humidities inside and outside the second container.

During this conditioning process, a group of plantlets may all be placed in a single container, or individual plantlets may be held in separate containers. Also, it is not necessary that the roots of the plantlets be covered with the potting mix during the conditioning process, but this is preferred because the potting mix helps to prevent the roots of the plantlets from drying as the plantlets become accustomed to the lower humidity.

One advantage of this invention is that, if desired, the culture medium can be changed or added as the plantlets grow. To add culture medium, the container 16 is opened, and the culture medium is simply added to the foam 12, for example, by pouring or spraying the medium on the foam, care being taken to avoid washing or splashing the embryos 18 and the plantlets from the foam. To change the culture medium, the foam and the plantlets are removed from the container, the old culture medium is removed from the foam, the foam and plantlets are then placed back in the container, and new culture medium is added. Here too, care should be taken to avoid accidentally washing the embryos and plantlets from the foam, both as the old culture medium is removed from and as the new culture medium is added to the foam.

With the present invention, a sufficient amount of the culture medium may be easily provided for the embryos and the plantlets to produce satisfactory growth. The culture medium can be regulated or replaced during embryo growth, without disturbing the foam or the embryos, in order to control the level of nutrients or other additives in the foam; and the culture medium can be easily removed from the foam before the plantlets are transplanted. The foam allows healthy root development and growth, yet offers excellent physical support for the plantlets during moving and planting. It was surprising that the open cell structure of the foam did not cause the embryos and the roots of the plantlets to dry out; and, in fact, the cell structure of the foam is of significant utility because it provides excellent, natural areation of the embryos and plantlets, and it is not necessary to perform any specific steps or to use any mechanical devices to areate the embryos and plantlets as they grow.

While it is apparent that the invention herein disclosed is well calculated to fulfill the objects previously stated, it will be appreciated that numerous modifications and embodiments may be devised by those skilled in the art, and it is intended that the appended claims cover all such modifications and embodiments as fall within the true spirit and scope of the present invention.

## Claims

1. A process for growing plantlets from embryos comprising:

providing a sterile growth environment including reticulated foam, a culture medium, and a container holding the foam and the culture medium;

inoculating the foam with the embryos; and

incubating the embryos under sterile, plantlet development conditions to develop plantlets from the embryos.

2. A process according to Claim 1 further comprising recovering the plantlets from the growth environment.

3. A process according to Claim 2 wherein the step of recovering the plantlets from the growth environment includes:

removing the foam and plantlets from the container; and

removing any remaining culture medium from the foam.

4. A process according to Claim 3 wherein:

the plantlets include roots; and

the step of recovering the plantlets from the growth environment further includes the step of covering the roots of the plantlets with a granular plant growth and support material.

5. A process according to Claims 2, 3 or 4 wherein:

the incubating step includes the step of growing the plantlets in an environment having a first humidity; and

the step of recovering the plantlets from the growth environment further includes the step of conditioning the plantlets to a second humidity after the roots of the plantlets are covered with the growth and support material.

6. A process according to Claims 1 -5 wherein the step of providing the sterile growth environment includes:

placing the foam in the container;

sterilizing the container and the foam; and

adding the culture medium to the foam.

7. A process according to Claim 6 wherein:

the culture medium is added to the foam after the container and the foam are sterilized; and

the culture medium is sterile when added to the foam.

8. A process according to claims 6 or 7 wherein:

the culture medium is a liquid; and

the culture medium is added to the foam prior to inoculating the foam with the embryos.

9. A process according to Claim 8 wherein the adding step includes thoroughly soaking the foam with the culture medium.

10. A process according to Claims 1 - 9 wherein the inoculating step includes the step of maintaining the embryos in an upper portion of the foam.

11. A process according to Claim 10 wherein the inoculating step includes the step of inoculating the embryos into the interior of the foam.

12. A process according to Claims 1 -11 wherein the incubating step includes the step of diffusing water vapor, carbon dioxide and oxygen through the container.

13. A process according to Claims 1 -12 wherein the incubating step includes the step of changing the culture medium.

14. Apparatus for growing plantlets from embryos comprising:

means providing a sterile growth environment including reticulated foam, a culture medium, and a container holding the foam and the culture medium;

a multitude of embryos inoculated onto the foam; and

means to incubate the embryos under sterile plantlet development conditions to grow plantlets from the embryos.

15. Apparatus according to Claim 14 wherein the culture medium is a liquid and the foam is thoroughly soaked with the culture medium.

16. Apparatus according to Claims 14 or 15 wherein the container includes a gas permeable cover to diffuse water vapor, carbon dioxide and oxygen through the container as the plantlets grow.

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | WO-A-8 604 919 (AGROGEN STIFTUNG)<br><br>* Abstract; page 3, line 15 - page 4, line 30; page 5, line 29 - page 6, line 5 * | 1,2,6, 12,14, 15 | A 01 G 7/00<br>A 01 G 31/00 |
| A | | 3,8-10 ,13,16 | |
| | --- | | |
| A,P | EP-A-0 171 593 (SUNGENE TECHNOLOGIES CORP.)<br>* Page 3, line 22 - page 4, line 10; page 4, line 28 - page 5, line 14; page 6, lines 3-19; claims 1,2 * | 1-5,12 ,13 | |
| | --- | | |
| A | GB-A-1 095 758 (MC DADE)<br><br>* Page 1, lines 27-73 * | 1-3,8, 13 | **TECHNICAL FIELDS SEARCHED (Int. Cl.4)** |
| | --- | | A 01 G<br>C 12 M |
| A | US-A-4 292 760 (KRAVE)<br><br>* Abstract; figure 1; column 3, line 25 - column 4, line 42; column 5, lines 7-36 * | 1,8,9, 16 | |
| | --- | | |
| A | US-A-4 299 921 (YOUSSEF)<br>* Figures; column 3, lines 5-51 * | 14,16 | |
| | --- | | |
| A | US-A-2 533 088 (BREWER et al.)<br>* Figures 1-3; column 1, line 54 - column 3, line 49 * | 14,16 | |
| | --- | | |

The present search report has been drawn up for all claims

| Place of search | Date of completion of the search | Examiner |
|---|---|---|
| THE HAGUE | 16-01-1987 | DISSEN H.D. |

EPO Form 1503 03 82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document